Europäisches Patentamt

European Patent Office

Office européen des brevets

(11) Publication number: **0 329 215**
**A1**

## (12) EUROPEAN PATENT APPLICATION

(21) Application number: **89200220.5**

(22) Date of filing: **02.02.89**

(51) Int. Cl.4: **C07C 126/02**

(30) Priority: **08.02.88 NL 8800284**

(43) Date of publication of application:
**23.08.89 Bulletin 89/34**

(84) Designated Contracting States:
**ES FR GB GR IT**

(71) Applicant: **STAMICARBON B.V.**
**Mijnweg 1**
**NL-6167 AC Geleen(NL)**

(72) Inventor: **Jonckers, Kees**
**Illikhoven 13**
**NL-6116 AK Susteren(NL)**

(54) **Process for the preparation of urea.**

(57) Process for the preparation of urea in which
- in a reaction zone a carbamate and free ammonia containing urea synthesis solution is formed from carbon dioxide and an excess of ammonia,
- in a stripping zone at least part of the carbamate that is present is decomposed with carbon dioxide as stripping gas,
- the carbamate decomposition products together with part of the excess of ammonia and the stripping gas are removed from the stripping zone as gas mixture,
- part of the gas mixture obtained is supplied to a condensation zone, in which it condenses, after which
- the carbamate containing mixture is supplied to the reaction zone and the stripped urea synthesis solution is processed into a urea solution or solid urea.

The carbamate containing solution is caused by gravity to flow out of the condensation zone to the reaction zone and from the reaction zone to stripping zone, placed at the same or virtually the same height, and the heat required for the formation of urea in the reaction zone is generated through condensation of at least part of the remainder of the gas mixture discharged from the stripping zone.

## PROCESS FOR THE PREPARATION OF UREA

The invention relates to a process for the preparation of urea from ammonia and carbon dioxide.

When ammonia and carbon dioxide are passed into a synthesis zone at a suitable pressure (for instance 125-350 at) and a suitable temperature (for instance 170-250°C), first ammonium carbamate is formed according to the reaction:

$$2\,NH_3 + CO_2 \rightarrow H_2N\text{-}CO\text{-}ONH_4$$

The ammonium carbamate thus formed gives urea through dehydration according to the equilibrium reaction:

$$H_2N\text{-}CO\text{-}ONH_4 \rightleftharpoons H_2N\text{-}CO\text{-}NH_2 + H_2O$$

The degree to which the conversion to urea proceeds depends on, among other factors, the temperature and the excess of ammonia used. The reaction product obtained is a solution mainly consisting of urea, water, ammonium carbamate and free ammonia. The ammonium carbamate and the ammonia have to be removed from the solution and are in most cases fed back to the synthesis zone.

A widely used mode of preparation of urea, known as "Stamicarbon carbon dioxide stripping process", is described for instance in European Chemical News, Urea Supplement, of January 17, 1969, pp.17-20 and in Nitrogen, No.143, of May-June 1983, pp. 32-38. In this process the urea synthesis solution formed in the synthesis zone at high pressure and temperature is subjected to a stripping treatment at synthesis pressure by contacting the solution counter-currently with gaseous carbon dioxide while supplying heat, causing the greater part of the carbamate present in the solution to be decomposed into ammonia and carbon dioxide. These decomposition products are expelled in gaseous form from the solution and discharged together with a minor quantity of water vapour and the carbon dioxide used for the stripping. The gas mixture obtained in the stripping process is substantially condensed in a condensation zone operated at synthesis pressure and absorbed in an aqueous solution obtained in the further treatment of the urea-containing solution, the condensation heat released being used for generation of steam or for heating of process flows.

For the purpose of creating an $NH_3/CO_2$ ratio that is optimum for the condensation, the greater part or all of the fresh ammonia required in the process is also supplied to the condensation zone. From the condensation zone, both the aqueous carbamate solution formed and the non-condensed gas mixture are supplied to the synthesis zone for formation of urea. Further condensation of the gas mixture here provides the heat required for the conversion of carbamate into urea.

The stripped urea synthesis solution is subsequently expanded to a low pressure and worked up to a urea solution or melt, which may be further processed to solid urea.

In the process described above the synthesis zone contains, besides the liquid phase, a gas phase consisting of relatively large quantities of ammonia, carbon dioxide as well as inert substances that were in part present as impurities in the reaction components, but which, if stainless steel equipment is used that is maintained in passivated condition by means of oxygen, may also have been introduced into the system as air. This gas mixture is removed from the synthesis zone, following which the entrained ammonia and carbon dioxide are recovered in a separation zone, for instance by washing the gases with a diluted carbamate solution. The inert residual gas is vented.

As is known, solutions containing ammonium carbamate are highly corrosive and erosive. For this reason the transport of such solutions in the high-pressure section of the urea synthesis is normally effected through natural circulation, which is achieved by placing the stripping zone below the level at which the synthesis zone and the condensation zone are situated. This eliminates the necessity of pumps, which would have to operate under extreme conditions and would be a source of many disorders. The liquid or dissolved ammonium carbamate formed in the separation zone for the inert gases is also circulated by means of gravity, either to the condensation zone or to the lower part of the synthesis zone. For this purpose the separation is situated at a higher level than the synthesis zone. With currently operated capacities, of 1000-1500 tonnes per day or more, this means that the height of the plant is considerable, in many cases at least 40 to 45 metres above ground level. This requires heavy supporting structures and replacement of equipment items is laborious and time consuming.

It has already been suggested (see US-A-4,208,347) that the preparation of urea can be carried out at high molar $NH_3/CO_2$ ratios in a two-section reaction zone, with the first section above the second, and with the discharge of the first section passing into the bottom part of the second section. From the upper part of the second section the urea synthesis solution is discharged and treated in two stripping zones in series for decomposition of carbamate, the gas phase obtained in the first stripping zone being as such passed directly into the first part of the reaction zone and the gas phase obtained in the second stripping zone, after condensation in a condensation zone, also being passed into the first part of the reaction zone as carbamate solution. The heat required in this first section is obtained by condensation of the gas mixture coming from the first stripping zone. In the second section the formation of urea is promoted by supplying an extra quantity of liquid $NH_3$, so that water is bound and the equilibrium is shifted to the right. According to the specification these measures avoid the necessity of space-taking, cumbersome scaffolds that are usually required to place the reactors higher than the equipment for treatment of the effluent from the synthesis. However, this embodiment is laborious and requires much equipment. Moreover, the reactor construction is complicated and therefore expensive.

The object of the invention is to provide a process with which the above-mentioned drawbacks are avoided and the "skyline" of the urea production plant is considerably improved, without the need of using a pump to transport the urea synthesis solution out of the reaction zone or the carbamate solution out of the condensation zone. This is achieved according to the invention through a combination of measures. For this purpose the condensation zone is placed at a level that is higher than the inlet of the urea synthesis solution to the stripping zone. The reaction zone and the stripping zone are placed at the same or virtually the same level, preferably at bottom level. The liquid phase is allowed to flow by gravity from the condensation zone via the reaction zone to the stripping zone, the driving force resulting from the difference in height between the level in the condensation zone and the urea synthesis solution inlet of the stripping zone. In addition, the heat required for the formation of urea in the reaction zone is generated by causing carbamate to be formed in this zone from part of the $NH_3$ and $CO_2$ containing gas mixture discharged out of the stripping zone.

The invention consequently relates to a process for the preparation of urea in which
- in a reaction zone at a pressure of 125-350 bar and the corresponding temperature of 170-250°C a carbamate and free ammonia containing urea synthesis solution is formed from carbon dioxide and an excess of ammonia at a molar $NH_3/CO_2$ ratio of 2.5-3.5,
- in a stripping zone at least part of the carbamate present in the urea synthesis solution is decomposed due to supply of heat and countercurrent contact with carbon dioxide as stripping gas,
- the carbamate decomposition products together with part of the excess of ammonia and the stripping gas are removed from the stripping zone as gas mixture,
- part of the gas mixture obtained is supplied to a condensation zone, in which it condenses to a carbamate containing mixture, after which
- the carbamate containing mixture is supplied to the reaction zone and the stripped urea synthesis solution is processed into a urea solution or solid urea.

The process is characterized in that the carbamate containing solution is caused by gravity to flow out of the condensation zone to the reaction zone and from the reaction zone to the stripping zone, placed at the same or virtually the same height, and the heat required for the formation of urea in the reaction zone is generated through condensation of part of the gas mixture discharged from the stripping zone.

Since the condensation zone, the reaction zone and the stripping zone are operated at the same pressure and the line connecting the condensation zone with the reaction zone as well as the line connecting the reacting zone with the stripping zone are filled with liquid, these equipment items will function as communicating vessels, with equal pressures at equal levels. If the outlet from the condensation zone is placed at a sufficient height above the inlet of the urea synthesis solution in the stripping zone, the liquid will flow by gravity from the condensation zone via the reaction zone to the stripping zone, in spite of the liquid level in the reaction zone being above the inlet in the stripping zone.

The heat required for the formation of urea in the reaction zone can be obtained in various ways, for instance by means of heat supply through a steam coil, which is difficult however in terms of construction, or by supplying a small portion of the $CO_2$ required for the process, which however affects the stripping efficiency in the stripping zone. A particularly suitable method is to supply to the reaction zone, and allow to condense therein, part of the $NH_3$, $CO_2$ and $H_2O$ containing gas mixture discharged from the stripping zone.

Since in the embodiment described the pressure in the bottom of the reaction zone is higher than in the top of the stripping zone, from where the gas mixture obtained in the stripping step is discharged, this gas mixture has to brought to a higher pressure prior to supplying it into the lower part of the reaction zone. This pressure increase will preferably be effected by means of an ejector, driven with the liquid $NH_3$ required in the process, since then no equipment with moving parts is subjected to the corrosive and erosive influence of the gas mixture.

At the relatively high pressures at which the system is operated the density of the gas mixture discharged from the stripping zone is relatively high, so that the behaviour of the gas mixture comes increasingly close to that of a liquid. It has been found that the entrainment effect of an ejector increases as the difference between the density of the driving medium and that of the medium to be put under a higher pressure becomes smaller. Moreover, with unchanged head and suction flow, less inlet pressure on the driving medium will be required.

According to a preferable embodiment the density of the liquid ammonia is lowered by heating it to above the critical temperature. Heating of the liquid ammonia can be effected for instance through heat exchange with the carbamate solution that is obtained when washing $NH_3$ and $CO_2$ out of the inert gases discharged from the synthesis zone and/or the condensation zone.

The portion of the gas mixture discharged from the stripping zone that is required for temperature control in the reaction zone is supplied into the bottom part of the compartmented reaction zone and led upwards therein, resulting in an even temperature evolution in the reaction zone. The quantity of gas mixture supplied to the reaction zone should be such that as a result of the condensation heat released the desired reaction temperature is reached. It may amount to for instance 15-25% of the total quantity discharged from the stripping zone. The quantities are not critical however. Any portion of the gas mixture that is not condensed in the reaction zone is led, together with the remaining portion from the stripping zone, into the condensation zone.

It is advantageous to cause urea formation from carbamate to take place as well in the condensation zone during the condensation of the gas mixture obtained in the stripping treatment. Due to the presence of relatively large amounts of urea and water, which act as solvents for the carbamate formed in the condensation of the gas mixture obtained during the stripping, the condensation and dissolution heat becomes available at a higher temperature level than without application of these solvents. This means that the condensation of the gas mixture obtained in the stripping treatment is carried at a temperature such that for dissipation of the heat released, with formation of low-pressure steam of for instance 3-5 bar, a considerably smaller heat-exchanging surface will suffice, steam of a higher pressure, for instance 5-10 bar, can be generated or the heat released can be used directly for the heating of process flows.

The process according to the invention offers the major advantage that the height of the plant is considerably lowered. Besides an improved "skyline" the installing and replacement costs are significantly reduced.

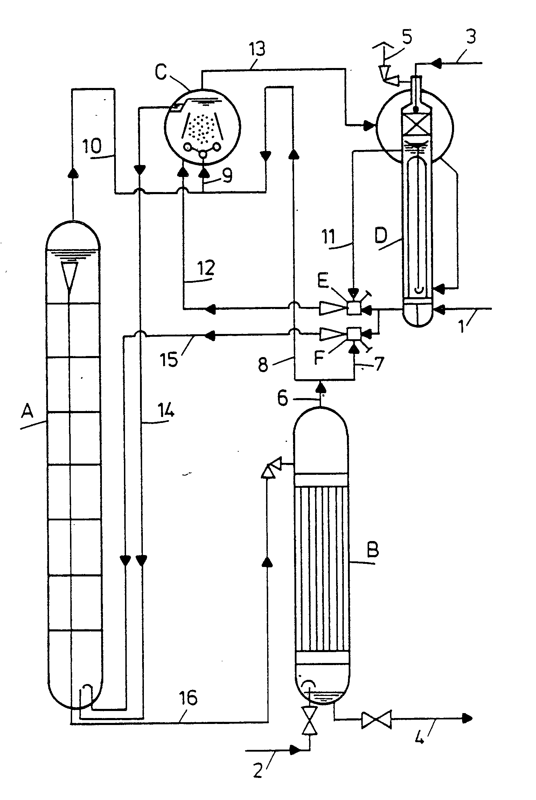

The invention will now be elucidated with reference to the figure and the example, without however being restricted thereto.

In the figure, A represents a reaction zone, B a stripping zone, C a condensation zone and D a separation zone. The pressure in these zones can be between 125 and 350 bar and amounts to for instance 140 bar. The amounts of $NH_3$ and $CO_2$ required in the process are supplied to these zones operated at high pressures. In addition, a carbamate solution obtained elsewhere in the process is recycled to this high-pressure section. The supply of the liquid $NH_3$ takes place via 1, of the $CO_2$ to be used as stripping gas via 2 and of the carbamate solution used for washing of $NH_3$ and $CO_2$ out of the inert gases via 3. To the condensation zone C is supplied, via 9, a quantity of gas mixture consisting of part of the gas mixture coming from the stripping zone via 6 and passed via 8 and the inert gases, still containing $NH_3$ and $CO_2$, discharged from the reaction zone via 10. Further, via 12, is supplied to the condensation zone the carbamate solution which is discharged from the separation zone D via 11 and which is obtained when washing the inert gases supplied via 13, which carbamate solution is passed into the condensation zone via 12, via ejector E, which is driven with part of the liquid $NH_3$ that is supplied via 1 and heated in the separation zone D. The drain valve for the inert gases is indicated by 5.

To the lower section of the reaction zone A is supplied, via 14, the carbamate solution formed in the condensation zone C, which carbamate solution flows upward in the compartmented reaction zone, resulting in formation of urea. The heat required for the formation of urea is generated through condensation of part of the $NH_3$ and $CO_2$ containing gas mixture obtained in stripping zone B and supplied to the lower part of the reaction zone via 6, 7, ejector F and 15. The pressure of the gas mixture is raised in ejector F, which is driven by hot liquid ammonia that has been heated to above its critical temperature, for instance 155°C, in order to overcome the resistance of the liquid head in the reaction zone. The urea synthesis solution in the synthesis zone via 16 flows by gravity into the top of the stripping zone B, which is placed at virtually the same height as the synthesis zone, into the lower part of which stripping zone the quantity of $CO_2$ required in the synthesis is introduced as stripping gas. The stripped urea synthesis solution is discharged via 4.

The invention will now be elucidated with the following embodiment example.

Example

In a urea production plant as schematically represented in the figure and having a capacity of 1000 tonnes per day, the reactor and the stripping facility are at ground level. The heights of the reactor and the stripper are about 18 metres and about 11 metres, respectively. On a floor situated 20 metres above ground level a horizontally mounted carbamate condenser is placed, which is of the submerged condenser design and has a diameter of about 3 metres. The section of the separation zone in which $NH_3$ and $CO_2$ are washed out of the inert gases is also placed at this level. The total height of the urea production plant is thus less than 25 metres.

The pressure of the installation amounts to 137.3 bar. The quantities are indicated in kg/h.

Via 1, 23,765 kg liquid $NH_3$ containing 71 kg water and having a temperature of 79.5⌐C and a pressure of 151.3 bar is passed into heat exchange in the separation zone D with the carbamate solution that is formed when $NH_3$ and $CO_2$ are washed out of the inert gases and has a temperature of 165⌐C. The temperature of the liquid $NH_3$ consequently rises to 155⌐C. Of this quantity, 16,760 kg is supplied to the ejector E, which via 11 draws in from the separation zone D 36,637 kg carbamate solution containing 13,462 kg $NH_3$, 14,057 kg $CO_2$, 2,126 kg urea, 6,946 kg $H_2O$ and 27 kg inert, after which the mixture is supplied to the condensation zone C via 12. To this condensation zone is also supplied, via 6, 8 and 9, a quantity of 76,382 kg gas mixture coming from the stripping zone B and containing 29,905 kg $NH_3$, 42,976 kg $CO_2$, 2,601 kg $H_2O$ and 901 kg inert. To the condensation zone C is further supplied, via 10, 7,751 kg gas mixture coming from the reaction zone A and containing 3,909 kg $NH_3$, 3,454 kg $CO_2$, 273 kg $H_2O$ and 115 kg inert. To the reaction zone A is supplied, via 14, 19,890 kg solution containing 37,095 kg $NH_3$, 28,448 kg $CO_2$, 35,024 kg urea, 19,308 kg $H_2O$ and 15 kg inert and having a temperature of 179⌐C. 8,777 kg gas mixture coming from the stripping zone B and having a temperature of 187⌐C is drawn in via the ejector F and passed into the reaction zone under a pressure of 139 bar. This gas mixture contains 3,436 kg $NH_3$, 4,938 kg $CO_2$, 299 kg $H_2O$ and 103 kg inert. The ejector is driven by 7,005 kg liquid $NH_3$ with a pressure of 151.3 bar and a temperature of 155⌐C. The temperature in the reaction zone is 186⌐C. Via 16, 127,921 kg urea synthesis solution, containing 45,195 kg urea, 145 kg biuret, 37,766 kg $NH_3$, 22,356 kg $CO_2$ and 22,456 kg $H_2O$ and 4 kg inert, is supplied to the stripping zone B, where it is stripped, while heat is supplied, with 31,800 kg $CO_2$ of 165⌐C, containing 137 kg $H_2O$ and 1,009 kg inert. Via 4, 74,562 kg stripped urea synthesis solution is obtained, containing 42,365 kg urea, 203 kg biuret, 6,002 kg $NH_3$, 7,120 kg $CO_2$, 18,864 kg $H_2O$ and 9 kg inert. For the stripping treatment, 785 kg high-pressure steam of 26 bar is required per tonne of urea produced. In the condensation zone 1,150 kg/t urea low-pressure steam of 4.5 bar is obtained.

## Claims

1. Process for the preparation of urea in which
- in a reaction zone at a pressure of 125-350 bar and the corresponding temperature of 170-250⌐C a carbamate and free ammonia containing urea synthesis solution is formed from carbon dioxide and an excess of ammonia at a molar $NH_3/CO_2$ ratio of 2.5-3.5,
- in a stripping zone at least part of the carbamate present in the urea synthesis solution is decomposed due to supply of heat and countercurrent contact with carbon dioxide as stripping gas,
- the carbamate decomposition products together with part of the excess of ammonia and the stripping gas are removed from the stripping zone as gas mixture,
- part of the gas mixture obtained is supplied to a condensation zone, in which it condenses to a carbamate containing mixture, after which
- the carbamate containing mixture is supplied to the reaction zone and the stripped urea synthesis solution is processed into a urea solution or solid urea,
characterized in that the carbamate containing solution is caused by gravity to flow out of the condensation zone to the reaction zone and from the reaction zone to stripping zone, placed at the same or virtually the same height, and the heat required for the formation of urea in the reaction zone is generated through condensation of at least part of the gas mixture discharged from the stripping zone.

2. Process according to claim 1, characterized in that the remaining part of the gas mixture discharged from the stripping zone is supplied into the lower part of the reaction zone by means of an ejector driven with ammonia in the supercritical state.

3. Process for the preparation of urea as described and elucidated with reference to the example and the figure.

4. Urea solutions or solid urea obtained through application of the process(es) according to claim(s) 1 and/or 2.

European Patent Office

**PARTIAL EUROPEAN SEARCH REPORT**
which under Rule 45 of the European Patent Convention shall be considered, for the purposes of subsequent proceedings, as the European search report

Application number

EP 89 20 0220

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 155 735 (UNIE VAN KUNSTMEST FABRIEKEN) <br> * Claims * | 1,4 | C 07 C 126/02 |
| A | FR-A-2 389 601 (MONTEDISON) <br> * Claims * <br> & US-A-4 208 347 (Cat. D) | 1,2,4 | |
| A | EP-A-0 132 194 (MONTEDISON) | | |

TECHNICAL FIELDS
SEARCHED (Int. Cl.4)

C 07 C 126/00

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2,4

Claims searched incompletely:

Claims not searched: 3

Reason for the limitation of the search:

See Rule 29.6 of the European Patent Convention

| Place of search | Date of completion of the search | Examiner |
|---|---|---|
| The Hague | 26-04-1989 | VAN GEYT |